Europäisches Patentamt

European Patent Office

Office européen des brevets

(19)

(11) **EP 1 204 871 B1**

(12) **EUROPÄISCHE PATENTSCHRIFT**

(45) Veröffentlichungstag und Bekanntmachung des Hinweises auf die Patenterteilung:
**21.04.2004 Patentblatt 2004/17**

(51) Int Cl.⁷: **G01N 33/86**, G01N 33/58, G01N 33/543

(21) Anmeldenummer: **00960351.5**

(22) Anmeldetag: **11.08.2000**

(86) Internationale Anmeldenummer:
**PCT/DE2000/002748**

(87) Internationale Veröffentlichungsnummer:
**WO 2001/013123 (22.02.2001 Gazette 2001/08)**

(54) **VERFAHREN ZUR INDIREKTEN BESTIMMUNG DES BLUTGERINNUNGSSTATUS**

METHOD FOR INDIRECTLY DETERMINING THE BLOOD-CLOTTING STATUS

PROCEDE POUR LA DETERMINATION INDIRECTE DE L'ETAT DE COAGULATION SANGUINE

(84) Benannte Vertragsstaaten:
**AT BE CH CY DE DK ES FI FR GB GR IE IT LI LU MC NL PT SE**

(30) Priorität: **14.08.1999 DE 19937654**
**31.08.1999 DE 19941447**

(43) Veröffentlichungstag der Anmeldung:
**15.05.2002 Patentblatt 2002/20**

(73) Patentinhaber: **november Aktiengesellschaft Gesellschaft für Molekulare Medizin**
**91056 Erlangen (DE)**

(72) Erfinder: **BERTLING, Wolf**
**D-91056 Erlangen (DE)**

(74) Vertreter: **Gassner, Wolfgang, Dr.**
**Patentanwalt,**
**Nägelsbachstrasse 49a**
**91052 Erlangen (DE)**

(56) Entgegenhaltungen:
WO-A-00/58732          WO-A-99/09058
DE-A- 4 008 546        US-A- 4 769 320
US-A- 4 780 410        US-A- 5 252 712

• **WEINSTOCK DAVID M ET AL: "Comparison of plasma prothrombin and factor VII and urine prothrombin F1 concentrations in patients on long-term warfarin therapy and those in the initial phase." AMERICAN JOURNAL OF HEMATOLOGY, Bd. 57, Nr. 3, März 1998 (1998-03), Seiten 193-199, XP002092275 ISSN: 0361-8609**

**Beschreibung**

Die Erfindung betrifft ein Verfahren zur indirekten Bestimmung des Blutgerinnungsstatus.

Die Bestimmung des Blutgerinnungsstatus kann indirekt durch Ermittlung der Prothrombinkonzentration in menschlichen Körperflüssigkeiten erfolgen. Bei Prothrombin handelt es sich um ein Protein, welches vorwiegend im Plasma des menschlichen Bluts vorkommt. Dieses Protein ist durch eine Vitamin-K abhänige γ-Carboxylase modifizierbar. Prothrombin ist mitverantwortlich für die Blutgerinnung. Es wandelt Fibrinogen in Fibrin um.

Die durch Prothrombin induzierte Umwandlung des Fibrinogens erfolgt nur dann, wenn Prothrombin in natürlicher carboxylierter Form vorliegt. Die Carboxylierung erfolgt in der Leber durch eine Carboxylase unter Bindung des Co-Faktors Vitamin K. Die Aktivität der Carboxylase ist von der Konzentration an Vitamin K abhängig. Bei reduzierter Aktivität der Carboxylase entsteht eine abnormale nicht carboxylierte Form des Prothrombins, welche nicht gerinnungsaktiv ist.

Beim gesunden Menschen liegt das Prothrombin in natürlicher, d.h. carboxylierter, Form vor. Die Carboxylierung wird durch Vitamin-K als Co-Faktor bewirkt. Bei kranken Menschen, insbesondere bei Menschen mit Leberschäden, oder bei Zugabe von Antikoagulantien, kommt Prothrombin auch in der abnormalen Form vor.

Das carboxylierte Prothrombin bewirkt eine Gerinnung nur dann, wenn zuvor $Ca^{2+}$-Ionen gebunden werden. Nur dann ist das carboxylierte Prothrombin in der Lage, an die Membranen der Blutplättchen zu binden und eine Gerinnung zu bewirken. Nur die carboxylierte Form des Prothrombins kann Calcium binden. Somit läßt der Gehalt an carboxyliertem Prothrombin auf den Blutgerinnungsstatus schließen.

Aus der US 4,769,320 ist ein Verfahren bekannt, bei dem die Ermittlung des Gehalts an carboxyliertem Prothrombin unter Verwendung von Antikörpern mittels Immunoassay erfolgt. Die Antikörper sind spezifisch für carboxyliertes Prothrombin in Gegenwart von Calcium. Sie binden nicht an decarboxyliertes Prothrombin. Es wird ein Kit zur Bestimmung des Pegels von carboxyliertem Prothrombin in einer Plasmaprobe beschrieben, der einen solchen Antikörper enthält.

Aus der US 5,252,712 ist ein monoklonaler Antiköper bekannt, der spezifisch für nicht-carboxyliertes Prothrombin ist. Unter Verwendung dieses Antikörpers läßt sich mittels Immunoassay die Konzentration an nicht-carboxyliertem Prothrombin ermitteln. Auch damit ist eine Aussage über den Blutgerinnungsstatus möglich.

In der US 4,780,410 ist ein Sandwich-Immunoassay-Verfahren zur Quantifizierung von einem decarboxylierten Prothrombin offenbart. Bei dem Verfahren wird ein gegen decarboxyliertes Prothrombin gerichteter immobilisierter monoklonaler Antikörper verwendet. Daran bindendes decarboxyliertes Prothrombin wird mittels eines zweiten gegen Prothrombin gerichteten Antikörpers detektiert. Es wird auch ein Kit zur Durchführung des Verfahrens beschrieben.

Aus Kornberg A. et al., Circulation 88 (1993), Seiten 454 - 460 ist es bekannt, die Konzentration von carboxyliertem Prothrombin in einer Probe mittels eines Kompetitors zu bestimmen. Dabei konkurriert Peroxidase-markiertes Prothrombin als Kompetitor mit dem Prothrombin in der Probe um die Bindung an einem immobilisierten Anti-Prothrombin-Antikörper. Das an dem Anti-Prothrombin-Antikörper gebundene Peroxidase-markierte Prothrombin ist mittels einer Enzymreaktion nachweisbar. Die Größe des dabei entstehenden Signals ist umgekehrt proportional zur Prothrombin-Konzentration in der Probe.

Aus der JP 05 284 994 A sind drei monoklonale Antikörper bekannt. Ein erster bindet spezifisch an humanes decarboxyliertes Prothrombin, ein zweiter spezifisch an humanes Prothrombin, humanes Thrombin und humanes decarboxyliertes Prothrombin und ein dritter spezifisch an humanes decarboxyliertes Prothrombin und humanes Prothrombin.

Aus von Kries, R. et al., Thrombosis and Haemostasis 68 (1992), Seiten 383 - 387 ist es bekannt, decarboxyliertes Prothrombin im Blut mittels eines ELISAs mit einem monoklonalen Antikörper zu bestimmen.

Nach dem Stand der Technik tritt das Problem auf, daß das zu analysierende Probenmaterial nicht immer unmittelbar nach der Entnahme der Probe analysiert wird. Durch die Versendung des Probenmaterials vergehen mitunter 1 bis 2 Tage. Die meisten der an der Blutgerinnung beteiligten Faktoren sind hoch empfindlich und schnell inaktiv. Während dieser Zeit wird u.a. sowohl carboxyliertes als auch nicht-carboxyliertes Prothrombin in der Probe abgebaut. Eine Verfälschung der Ergebnisse des Blutgerinnungsstatus ist die Folge.

Aufgabe der Erfindung ist es, die Nachteile nach dem Stand der Technik zu beseitigen. Es soll insbesondere die Genauigkeit der Ermittlung des Blutgerinnungsstatus mittels der Bestimmung des Prothrombingehalts erhöht werden. Ferner soll ein Kit bereitgestellt werden, der eine genauere Ermittlung des Blutgerinnungsstatus ermöglicht.

Diese Aufgabe wird durch die Merkmale der Ansprüche 1, 11 und 15 gelöst. Zweckmäßige Ausgestaltungen der Erfindung ergeben sich aus den Merkmalen der Ansprüche 2 bis 10 und 12 bis 20.

Nach Maßgabe der Erfindung ist ein Verfahren zur indirekten Bestimmung des Blutgerinnungsstatus mit folgenden Schritten vorgesehen:

a) Bereitstellen einer Körperflüssigkeitsprobe, die ein durch eine Vitamin-K abhängige γ-Carboxylase modifizierbares Protein enthält,

b) Ermittlung von mindestens zwei Konzentrationen, ausgewählt aus einer Gruppe bestehend aus einer ersten Konzentration an carboxyliertem Protein, einer zweiten Konzentration an decarboxyliertem Protein und einer Gesamtkonzentration an carboxyliertem und decarboxyliertem Protein, wobei die erste Konzentration unter Verwendung eines ersten Antikörpers, die zweite Konzentration unter Verwendung eines zweiten Antikörpers und die dritte Konzentration unter Verwendung eines dritten Antikörpers ermittelt wird,

c) Bildung eines ersten Quotienten aus erster und zweiter Konzentration
oder
Bildung eines zweiten Quotienten aus dritter und erster Konzentration
oder
Bildung eines dritten Quotienten aus dritter und zweiter Konzentration,
wobei eine zur Bildung des ersten, zweiten oder dritten Quotienten erforderliche und bei Schritt lit. b) nicht ermittelte Konzentration gemäß folgender Beziehung:

$$C3 - C2 = C1$$

errechnet wird
und

d) Korrelation des ersten, zweiten oder dritten Quotienten mit dem Blutgerinnungsstatus.

Unter einem durch eine Vitamin-K abhängige γ-Carboxylase modifizierbaren Protein wird ein Protein verstanden, das in Abhängigkeit des Blutgerinnungsstatus anteilig sowohl in caboxylierter als auch in decarboxylierter Form vorliegen kann. Das Protein kann ein leicht von einem Patienten gewinnbares Protein, z.B. ein Protein aus dem Speichel, sein. Das Protein kann ein Protein sein, das derselben prozentualen Hypomodifikation unterliegt wie Prothrombin. Ein Antikörper im Sinne der Erfindung kann ein Antikörper, ein Antikörperfragment oder ein sonstiger Stoff mit Bindungsspezifität für die carboxylierte Form, die decarboxylierte Form oder beiden Formen des Proteins sein.

Mit den erfindungsgemäßen Verfahren ist es möglich, auf der Basis des Gehalts an modifizierbarem Protein den Blutgerinnungsstatus genau zu ermitteln. Durch die Betrachtung sowohl des Gehalts an carboxyliertem Protein als auch des Gehalts an decarboxyliertem Protein und das Inbeziehungsetzen der beiden vorgenannten Proteingehalte werden Fehler bei der Bestimmung des Blutgerinnungsstatus minimiert.

In einer Ausführungsform der Erfindung wird beim Schritt lit. b) zusätzlich mindestens ein erster Kompetitor zur Ermittlung der ersten Konzentration, ein zweiter Kompetitor zur Ermittlung der zweiten Konzentration oder ein dritter Kompetitor zur Ermittlung der dritten Konzentration verwendet. Bei dem Kompetitor handelt es sich um einen Stoff, der mit dem carboxylierten Protein, dem decarboxylierten Protein oder dem carboxylierten und dem decarboxylierten Protein um die Bindung an einem der Antikörper konkurriert. Der Kompetitor kann das carboxylierte oder decarboxylierte Protein sein, wobei es mit einer Markierungssubstanz versehen ist. Anstatt des vollständigen Proteins kann auch ein Fragment dieses Proteins als Kompetitor verwendet werden.

Vorzugsweise ist mindestens einer der Antikörper oder mindestens einer der Kompetitoren mit einer Markierungssubstanz, insbesondere einem Enzym, einem Fluoreszenz-Farbstoff, einem Quencher, einem Goldpartikel, einem Latexpartikel, Biotin, Streptavidin oder Avidin, konjugiert. Als Enzym kann jedes Enzym verwendet werden, das mittels einer enzymatischen Reaktion nachgewiesen werden kann. Die Markierung des Antikörpers mit einem Goldpartikel erlaubt den Nachweis des gebundenen Antikörpers mittels eines Plasmonresonanz-Verfahrens. Anstatt der Ermittlung der mindestens zwei Konzentrationen gemäß Schritt lit. b) kann auch ein dazu korrelierendes Mischsignal unter Verwendung von zwei Antikörpern, ausgewählt aus einer Gruppe bestehend aus dem ersten, dem zweiten und dem dritten Antikörper, und gegebenenfalls mindestens einem der Kompetitoren erzeugt und ermittelt und direkt mit dem Blutgerinnungsstatus korreliert werden. Das Mischsignal entsteht durch das Zusammenwirken unterschiedlicher Einzelsignale. Es entspricht dem ersten, zweiten oder dritten Quotienten. Die Bildung dieser Quotienten gemäß Schritt lit. c) entfällt. Das Verfahren ist dadurch schneller und einfacher ausführbar. Das Mischsignal kann eine, insbesondere durch Fluoreszenzfarbstoffe erzeugte, Mischfarbe, ein durch den Förster-Effekt hervorgerufenes Fluoreszenzsignal oder eine durch den Quencher bedingte Verminderung eines Fluoreszenzsignals sein. Die Mischfarbe kann auch durch zwei Enzyme erzeugt werden, die jeweils eine spezifische Farbreaktion katalysieren. Bei dem Förster-Effekt findet ein strahlungsloser Energietransfer von einem angeregten ersten Fluorophor auf ein unmittelbar benachbartes zweites Fluorophor statt. Dadurch geht das erste Fluorophor in den Grundzustand über, während das zweite Fluorophor angeregt wird und fluoresziert. Bei dem erfindungsgemäßen Verfahren können z. B. die ersten und zweiten Antikörper mit Fluorophoren konjugiert sein, die den Förster-Effekt ermöglichen. Die Bindung der ersten Antikörper in unmittelbarer Nachbarschaft zu den zweiten Antikörpern kann mittels eines durch den Förster-Effekt hervorgerufenen Fluoreszenzsignals festgestellt werden. Bei einem strahlungslosen Energietransfer vom Fluorophor auf den Quencher findet eine Löschung der Fluoreszenz statt. Das insgesamt meßbare Fluoreszenzsignal wird dadurch vermindert.

Bei der Körperflüssigkeit kann es sich zweckmäßigerweise um Plasma, Blut, Speichel, Urin oder dgl.

handeln. Geeignet sind grundsätzlich alle Körperflüssigkeiten, in der das modifizierbare Protein in einem Gehalt enthalten ist, der eine Messung ermöglicht.

Nach einem Ausgestaltungsmerkmal der Erfindung erfolgt die Ermittlung der ersten, zweiten und/oder dritten Konzentration oder des Mischsignals mittels eines immunologischen Verfahrens. Dabei kann bei dem immunologischen Verfahren mindestens einer der Antikörper auf einem Träger, insbesondere einem Kunststoff, einem Magnetpartikel, einem Latexpartikel, einem Goldpartikel, einem Teststreifen oder einer Membran, immobilisiert sein. Der Kunststoff kann in Form eines Teströhrchens, eines Teststreifens, eines Kunststoffpartikels oder einer Vertiefung einer Mikrotiterplatte vorliegen.

Die erste, zweite und/oder dritte Konzentration und/oder das Mischsignal kann mittels einer Farbreaktion oder Fluoreszenzdetektion ermittelt werden. Damit ist eine besonders schnelle und einfache Ermittlung des Blutgerinnungsstatus möglich.

Bei dem durch eine Vitamin-K abhängige γ-Carboxylase modifizierbaren Protein handelt es sich vorzugsweise um einen der in der decarboxylierten Form als "Proteins Induced by Vitamin K Antagonism or Absence" (PIVKA-Faktoren) bezeichneten Gerinnungsfaktoren Prothrombin, Faktor VII, Faktor IX oder Faktor X, um Nephrocalcin oder um Osteocalcin. Es ist auch möglich, zur Bestimmung des Blutgerinnungsstatus andere Proteine zu benutzen, die von einer Vitamin-K abhängigen Carboxylase carboxyliert werden und ebenfalls mittels oral verabreichbarer Anticoagulantien beeinflußbar sind. Nephrocalcin ist z.B. im Urin nachweisbar. Es muß bei Benutzung dieses Proteins kein Blut entnommen werden. Das bedeutet für Patienten, deren Blutgerinnungsstatus laufend überwacht werden muß, eine erhebliche Erleichterung.

Es ist ferner ein Kit zur Durchführung des erfindungsgemäßen Verfahrens vorgesehen, wobei mindestens zwei Antikörper enthalten sind, ausgewählt aus einer Gruppe bestehend aus einem ersten Antikörper zur immunologischen Bestimmung einer ersten Konzentration der carboxylierten Form des Proteins, einem zweiten Antikörper zur immunologischen Bestimmung einer zweiten Konzentration der decarboxylierten Form des Proteins und einem dritten Antikörper zur immunologischen Bestimmung einer Gesamtkonzentration an carboxyliertem und decarboxyliertem Protein. Der erste und zweite Antikörper ist jeweils mit einer Markierungssubstanz konjugiert, wobei die Markierungssubstanzen so gewählt sind, daß sie gemeisam ein Mischsignal erzeugen können.

Es kann sich beim ersten, zweiten und dritten Antikörper um nach dem Stand der Technik bekannte Antikörper handeln. Solche Antikörper sind z.B. aus der US 5,252,712 und US 4,769,320 bekannt, deren Inhalt hiermit in die Beschreibung einbezogen wird.

In dem Kit kann zusätzlich mindestens ein erster Kompetitor zur Ermittlung der ersten Konzentration, ein zweiter Kompetitor zur Ermittlung der zweiten Konzentration oder ein dritter Kompetitor zur Ermittlung der dritten Konzentration enthalten sein. Mindestens einer der in dem Kit enthaltenen Antikörper oder Kompetitoren kann mit einer Markierungssubstanz, insbesondere einem Enzym, einem Fluoreszenz-Farbstoff, einem Quencher, einem Goldpartikel, einem Latexpartikel, Biotin, Streptavidin oder Avidin; konjugiert sein.

Vorzugsweise sind der erste und der zweite Antikörper auf einem Träger immobilisiert. Der Träger kann ein Kunststoff, ein Magnetpartikel, ein Latexpartikel, ein Goldpartikel, ein Teststreifen oder eine Membran sein. Ist der Träger ein Teststreifen, können der erste und der zweite Antikörper jeweils auf einem separaten Feld des Teststreifens aufgenommen sein. Vorzugsweise ist in dem Kit ein mit einer Markierungssubstanz, insbesondere einem Enzym, einem Fluoreszenz-Farbstoff, einem Quencher, einem Goldpartikel, einem Latexpartikel, Biotin, Streptavidin oder Avidin, konjugierter dritter Antikörper enthalten. Das ermöglicht eine besonders einfache Ermittlung des jeweiligen Gehalts z.B. mittels einer Farbreaktion auf dem Teststreifen.

In einer weiteren Ausgestaltung der Erfindung ist der dritte Antikörper auf dem oder einem weiteren Träger, insbesondere einem Kunststoff, einem Magnetpartikel, einem Latexpartikel, einem Goldpartikel, einem Teststreifen oder einer Membran, immobilisiert. Ist der Träger der oder ein weiterer Teststreifen, kann der dritte Antikörper auf einem Feld des Teststreifens aufgenommen sein. Bevorzugt sind in dem Kit mit jeweils einer Markierungssubstanz, insbesondere einem Enzym, einem Fluoreszenz-Farbstoff oder einem Quencher, konjugierte erste und zweite Antikörper enthalten, wobei die Markierungssubstanzen so gewählt sind, daß sie gemeinsam ein Mischsignal, insbesondere eine Mischfarbe, ein durch den Förster-Effekt hervorgerufenes Fluoreszenzsignal oder eine durch einen Quencher bedingte Verminderung eines Fluoreszenzsignals, erzeugen können. Das Mischsignal entspricht dem ersten Quotienten.

Bei dem Protein handelt es sich vorzugsweise um Prothrombin, Faktor VII, Faktor IX, Faktor X, Nephrocalcin oder Osteocalcin. Nachfolgend wird das erfindungsgemäße Verfahren anhand der Zeichnung erläutert: Es zeigen

Fig. 1      die Korrelation des Blutgerinnungsstatus mit cPT/dcPT,

Fig. 2      die Korrelation des Blutgerinnungsstatus mit dcPT/cPT und

Fig. 3      die Korrelation des Blutgerinnungsstatus mit dcPT.

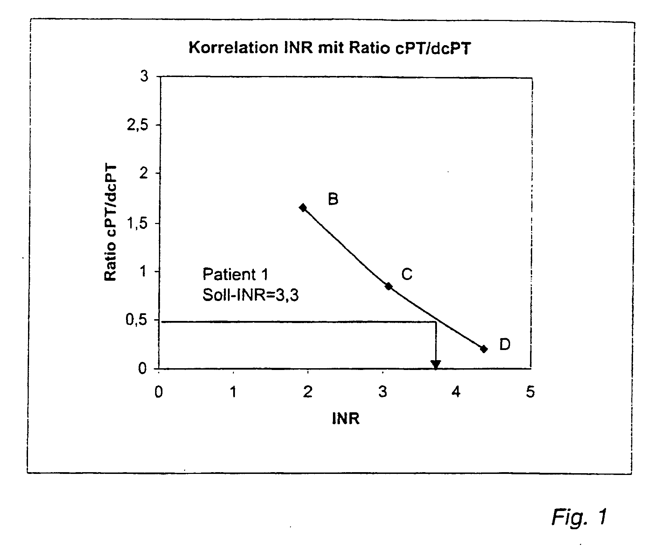

In Fig. 1 ist der Quotient aus den Konzentrationen von carboxyliertem und decarboxyliertem Prothrombin über dem Blutgerinnungsstatus INR aufgetragen. Die Konzentrationen sind hier als OD-Werte gemessen. Bei einem ermittelten Quotienten von 0,5 ergibt sich ein Blutgerinnungsstatus INR von 3,8.

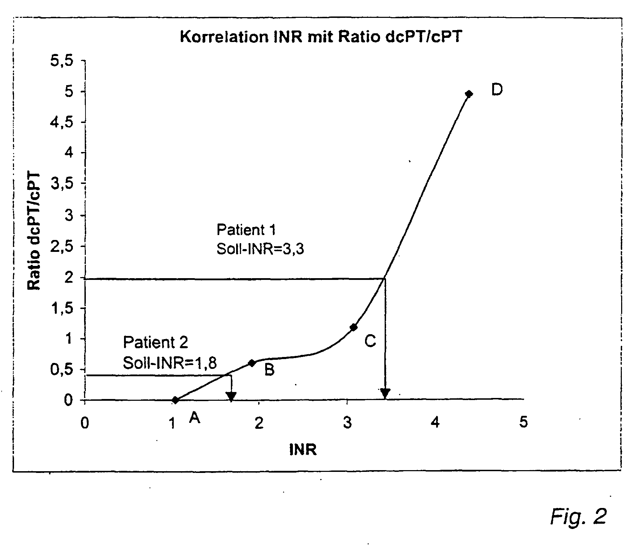

In Fig. 2 ist der Quotient aus den Konzentrationen von decarboxyliertem und carboxyliertem Prothrombin über dem Blutgerinnungsstatus aufgetragen. Es ist ersichtlich, daß der Quotient hier besonders gut mit dem Blutgerinnungsstatus INR korreliert.

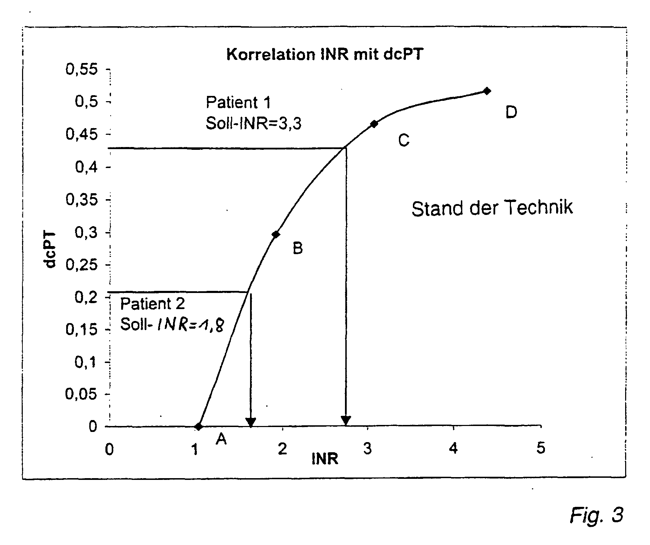

Fig. 3 zeigt die nach dem Stand der Technik bekannte Korrelation von dcPT mit dem Blutgerinnungsstatus INR. Diese verändert sich mit zunehmendem Alter der Proben.

Beispiel 1:

Für Serienmessungen besonders geeignet ist der sogenannte ELISA auf einer Mikrotiterplatte.

a) Probenvorbereitung:

Die Kavitäten einer Mikrotiterplatte (Maxisorb, NUNC) werden über Nacht bei 4°C mit je 50μl eines Antikörpers (10μg/ml in Carbonatpuffer) beschichtet. Die Kavitäten werden dreimal mit PBS gewaschen. Unspezifische Bindungsstellen werden eine Stunde bei Zimmertemperatur mit 50μl 1% BSA in PBS pro Kavität abgesättigt. Anschließend werden die Kavitäten dreimal mit PBS/0,05% Tween 20 gewaschen.

Anschließend werden die folgenden jeweils 1:50 in PBS/0,1% BSA verdünnten Proben aufgetragen (50μl/Kavität):

Kalibrierplasmen,
Normalplasma,
Patientenplasma und
Prothrombindefizientes Plasma (negative Kontrolle).

Die Mikrotiterplatte wird eine Stunde bei Zimmertemperatur inkubiert. Anschließend werden die Kavitäten dreimal mit PBS/0,05% Tween 20 gewaschen. Es werden 50μl/Kavität Kaninchen Anti-Gesamt-Prothrombin (10μg/ml) zufügt. Dann wird die Mikrotiterplatte eine Stunde bei Zimmertemperatur inkubiert. Die Kavitäten werden anschließend dreimal mit PBS/0,05% Tween 20 gewaschen. Es werden 50μl/Kavität Ziege-anti-Kaninchen-Antikörper, Biotin-konjugiert (Dianova, 1:20000 in PBS/0,1% BSA), zufügt. Die Mikrotiterplatte wird eine Stunde bei Zimmertemperatur inkubiert. Anschließend werden die Kavitäten dreimal mit PBS/0,05% Tween 20 gewaschen.

Es werden 50μl/Kavität Streptavidin-Peroxidase-Konjugat (Roche Diagnostics, 1:1000 in Konjugatpuffer) zufügt. Die Mikrotiterplatte wird eine Stunde bei Zimmertemperatur inkubiert. Anschließend werden die Kavitäten dreimal mit PBS/0,05% Tween 20 gewaschen.

Zur Durchführung der Entwicklungsreaktion werden 50μl/Kavität ABTS-Lösung (Roche Diagnostics, 1mg/ml) zufügt. Die Mikrotiterplatte wird eine halbe bis eine Stunde bei Zimmertemperatur inkubiert. Die Absorbtionswerte (OD-Werte) werden in einem ELISA-Reader gemessen.

Es versteht sich, daß das Verfahren erheblich verkürzt werden kann, wenn bereits der zur Detektion gebundenen Prothrombins verwendete Anti-Prothrombin-Antikörper eine Markierungssubstanz, wie Peroxidase oder ein anderes Enzym, aufweist. Eine weitere Verkürzung des Verfahrens kann durch Verwendung einer direkt detektierbaren Markierungssubstanz, wie einem Fluorophor, erreicht werden. Bei Verwendung einer solchen Markierungssubstanz ist keine Entwicklungsreaktion erforderlich.

b) Auswertung:

Es werden

aa) die OD-Werte des Gesamt-Prothrombins (Kavitäten sind mit monoklonalen Anti-Gesamt-Prothrombin-Antikörpern beschichtet),

bb) die OD-Werte des decarboxylierten Prothrombins (Kavitäten sind mit monoklonalen Anti-Decarboxy-Prothrombin-Antikörpern beschichtet) und

cc) die OD-Werte des carboxylierten Prothrombins (Differenz zwischen den OD-Werten des Gesamt-Prothrombins und den OD-Werten des decarboxylierten Prothrombins)

bestimmt.

Dann werden Eichkurven aus den gemessenen OD-Werten (siehe Fig. 1 - 3: Punkte A,B,C und D) der Kalibrierplasmen erstellt und der INR der Patientenplasmen berechnet.

Es versteht sich, daß der Gerinnungsstatus auch durch Ermittlung der entsprechenden Konzentrationen anderer durch eine Vitamin-K abhängige γ-Carboxylase modifizierbarer Proteine als Prothrombin ermittelt werden kann. Solche Proteine sind z.B. Faktor VII, Faktor IX, Faktor X, Nephrocalcin oder Osteocalcin.

Beispiel 2:

Die Kavitäten einer Mikrotiterplatte (Maxisorb, NUNC) werden über Nacht bei 4°C mit je 50μl eines gegen carboxyliertes und decarboxyliertes Prothrombin oder eines nur gegen decarboxyliertes Prothrombin gerichteten Antikörpers (10μg/ml in Carbonatpuffer) beschichtet. Die Kavitäten werden dreimal mit PBS gewaschen. Unspezifische Bindungsstellen werden eine Stunde bei Zimmertemperatur mit 50μl 1% BSA in PBS pro Kavität abgesättigt. Anschließend werden die Kavitäten dreimal mit PBS/0,05% Tween 20 gewaschen.

Peroxidase-markiertes decarboxyliertes Prothrombin wird zusammen mit den folgenden jeweils 1: 50 in PBS/0,1% BSA verdünnten Proben in einer Endkonzentrationen von 30 µg/ml in die Antikörper-beschichteten Kavitäten der Mikrotiterplatte gegeben (50µl/Kavität):

Kalibrierplasmen,
Normalplasma,
Patientenplasma und
Prothrombindefizientes Plasma (negative Kontrolle).

Die Mikrotiterplatte wird eine Stunde bei Zimmertemperatur inkubiert. Anschließend werden die Kavitäten dreimal mit PBS/0,05% Tween 20 gewaschen. Es werden 50µl/Kavität ABTS-Lösung (Roche Diagnostics, 1mg/ml) zugefügt. Die Mikrotiterplatte wird eine halbe bis eine Stunde bei Zimmertemperatur inkubiert. Die OD-Werte in den Kavitäten der Mikrotiterplatte werden im ELISA-Reader gemessen. Je höher der OD-Wert in einer Kavität ist, desto geringer ist die Konzentration des Prothrombins in der jeweiligen Probe. Die Prothrombinkonzentration im Patientenplasma wird aufgrund einer mittels der Kalibrierplasmen erstellten Eichkurve ermittelt. Mit dem Antikörper gegen carboxyliertes und decarboxyliertes Prothrombin beschichtete Kavitäten werden zur Bestimmung der Gesamtkonzentration des carboxylierten und decarboxylierten Prothrombins verwendet. Kavitäten, die mit dem gegen decarboxyliertes Prothrombin gerichteten Antikörper beschichtet sind, dienen zur Bestimmung der Konzentration an decarboxyliertem Prothrombin. Die ermittelte Gesamtkonzentrationen an carboxyliertem und decarboxyliertem und die Konzentration an decarboxyliertem Prothrombin werden zueinander ins Verhältnis gesetzt. Aus dem resultierenden Quotienten kann anhand der Quotienten für die Kalibrierplasmen der Gerinnungsstatus ermittelt werden.

**Patentansprüche**

1. Verfahren zur indirekten Bestimmung des Blutgerinnungsstatus mit folgenden Schritten:

a) Bereitstellen einer Körperflüssigkeitsprobe, die ein durch eine Vitamin-K abhängige γ-Carboxylase modifizierbares Protein enthält,

b) Ermittlung von mindestens zwei Konzentrationen, ausgewählt aus einer Gruppe bestehend aus einer ersten Konzentration (C1) an carboxyliertem Protein, einer zweiten Konzentration (C2) an decarboxyliertem Protein und einer Gesamtkonzentration (C3) an carboxyliertem und decarboxyliertem Protein, wobei die erste Konzentration (C1) unter Verwendung eines ersten Antikörpers (A1), die zweite Konzentration (22) unter Verwendung eines zweiten Antikörpers (A2) und die dritte Konzentration (C3) unter Verwendung eines dritten Antikörpers (A3) ermittelt wird,

c) Bildung eines ersten Quotienten (Q1) aus erster (C1) und zweiter Konzentration (C2)
oder
Bildung eines zweiten Quotienten (Q2) aus dritter (C3) und erster Konzentration (C1)
oder
Bildung eines dritten Quotienten (Q3) aus dritter (C3) und zweiter Konzentration (C2),
wobei eine zur Bildung des ersten (Q1), zweiten (Q2) oder dritten Quotienten (Q3) erforderliche und bei Schritt lit. b) nicht ermittelte Konzentration (C1, C2, C3) gemäß folgender Beziehung:

$$C3 - C2 = C1$$

errechnet wird
und

d) Korrelation des ersten, zweiten oder dritten Quotienten (Q1, Q2, Q3) mit dem Blutgerinnungsstatus.

2. Verfahren nach Anspruch 1, wobei beim Schritt lit. b) zusätzlich mindestens ein erster Kompetitor (K1) zur Ermittlung der ersten Konzentration (C1), ein zweiter Kompetitor (K2) zur Ermittlung der zweiten Konzentration (C2) oder ein dritter Kompetitor (K3) zur Ermittlung der dritten Konzentration (C3) verwendet wird.

3. Verfahren nach Anspruch 1 oder 2, wobei mindestens einer der Antikörper (A1, A2, A3) oder mindestens einer der Kompetitoren (K1, K2,K3) mit einer Markierungssubstanz, insbesondere einem Enzym, einem Fluoreszenz-Farbstoff, einem Quencher, einem Goldpartikel, einem Latexpartikel, Biotin, Streptavidin oder Avidin, konjugiert ist.

4. Verfahren nach einem der vorhergehenden Ansprüche, wobei anstatt der Ermittlung der mindestens zwei Konzentrationen gemäß Schritt lit. b) ein dazu korrelierendes Mischsignal unter Verwendung von zwei Antikörpern, ausgewählt aus einer Gruppe bestehend aus dem ersten (A1), dem zweiten (A2) und dem dritten Antikörper (A3), und gegebenenfalls mindestens einem der Kompetitoren (K1, K2, K3) erzeugt und ermittelt und direkt mit dem Blutgerinnungsstatus korreliert wird.

5. Verfahren nach Anspruch 4, wobei das Mischsignal eine, insbesondere durch Fluoreszenzfarbstoffe erzeugte, Mischfarbe, ein durch den Förster-Effekt hervorgerufenes Fluoreszenzsignal oder eine durch den Quencher bedingte Verminderung eines Fluoreszenzsignals ist.

6. Verfahren nach einem der vorhergehenden Ansprüche, wobei die Körperflüssigkeit Plasma, Blut, Speichel, Urin oder dgl. ist.

7. Verfahren nach einem der vorhergehenden Ansprüche, wobei die Ermittlung der ersten (C1), zweiten (C2) und/oder dritten Konzentration (C3) oder des Mischsignals mittels eines immunologischen Verfahrens erfolgt.

8. Verfahren nach Anspruch 7, wobei bei dem immunologischen Verfahren mindestens einer der Antikörper (A1, A2, A3) auf einem Träger, insbesondere einem Kunststoff, einem Magnetpartikel, einem Latexpartikel, einem Goldpartikel, einem Teststreifen oder einer Membran, immobilisiert ist.

9. Verfahren nach einem der vorhergehenden Ansprüche, wobei die erste (C1), zweite (C2) und/oder dritte Konzentration (C3) und/oder das Mischsignal mittels einer Farbreaktion oder Fluoreszenzdetektion ermittelt wird.

10. Verfahren nach einem der vorhergehenden Ansprüche, wobei das durch eine Vitamin-K abhängige γ-Carboxylase modifizierbare Protein Prothrombin, Faktor VII, Faktor IX, Faktor X, Nephrocalcin oder Osteocalcin ist.

11. Kit zur Durchführung des Verfahrens nach einem der vorhergehenden Ansprüche, mit einem ersten Antikörper (A1) zur immunologischen Bestimmung einer ersten Konzentration (C1) der carboxylierten Form des Proteins und einem zweiten Antikörper (A2) zur immunologischen Bestimmung einer zweiten Konzentration (C2) der decarboxylierten Form des Proteins,
**dadurch gekennzeichnet, daß**
erste (A1) und zweite Antikörper (A2) jeweils mit einer Markierungssubstanz konjugiert ist, wobei die Markierungssubstanzen so gewählt sind, daß sie gemeinsam ein Mischsignal erzeugen können.

12. Kit nach Anspruch 11, wobei die Markierungssubstanz ein Enzym, ein Fluoreszenz-Farbstoff oder ein Quencher ist.

13. Kit nach Anspruch 11 oder 12, wobei das Mischsignal eine Mischfarbe, ein durch den Förster-Effekt hervorgerufenes Fluoreszenzsignal oder eine durch einen Quencher bedingte Verminderung eines Fluoreszenzsignals ist.

14. Kit nach einem der Ansprüche 11 bis 13, wobei das Protein Prothrombin, Faktor VII, Faktor IX, Faktor X, Nephrocalcin oder Osteocalcin ist.

15. Verwendung eines Kits,
enthaltend mindestens zwei Antikörper, ausgewählt aus einer Gruppe bestehend aus einem ersten Antikörper (A1) zur immunologischen Bestimmung einer ersten Konzentration (C1) der carboxylierten Form des Proteins, einem zweiten Antikörper (A2) zur immunologischen Bestimmung einer zweiten Konzentration (C2) der decarboxylierten Form des Proteins und einem dritten Antikörper (A3) zur immunologischen Bestimmung einer Gesamtkonzentration (C3) an carboxyliertem und decarboxyliertem Protein,
zur Durchführung des Verfahrens nach einem der Ansprüche 1 bis 10.

16. Verwendung nach Anspruch 15, wobei zusätzlich mindestens ein erster Kompetitor (K1) zur Ermittlung der ersten Konzentration (C1), ein zweiter Kompetitor (K2) zur Ermittlung der zweiten Konzentration (C2) oder ein dritter Kompetitor (K3) zur Ermittlung der dritten Konzentration (C3) enthalten ist.

17. Verwendung nach Anspruch 15 oder 16, wobei mindestens einer der enthaltenen Antikörper (A1, A2, A3) oder Kompetitoren (K1, K2, K3) mit einer Markierungssubstanz, insbesondere einem Enzym, einem Fluoreszenz-Farbstoff, einem Quencher, einem Goldpartikel, einem Latexpartikel, Biotin, Streptavidin oder Avidin, konjugiert ist.

18. Verwendung nach einem der Ansprüche 15 bis 17, wobei der erste (A1) und der zweite Antikörper (A2) auf einem Träger, insbesondere einem Kunststoff, einem Magnetpartikel, einem Latexpartikel, einem Goldpartikel, einem Teststreifen oder einer Membran, immobilisiert sind.

19. Verwendung nach Anspruch 18, wobei der Träger ein Teststreifen ist und der erste (A1) und der zweite Antikörper (A2) jeweils auf einem separaten Feld des Teststreifens aufgenommen sind.

20. Verwendung nach Anspruch 18 oder 19, wobei ein mit einer Markierungssubstanz, insbesondere einem Enzym, einem Fluoreszenz-Farbstoff, einem Quencher, einem Goldpartikel, einem Latexpartikel, Biotin, Streptavidin oder Avidin, konjugierter dritter Antikörper (A3) enthalten ist.

21. Verwendung nach einem der Ansprüche 15 bis 20, wobei der dritte Antikörper (A3) auf dem oder einem weiteren Träger, insbesondere einem Kunststoff, einem Magnetpartikel, einem Latexpartikel, einem Goldpartikel, einem Teststreifen oder einer Membran, immobilisiert ist.

22. Verwendung nach Anspruch 21, wobei der Träger der oder ein weiterer Teststreifen ist und der dritte Antikörper (A3) auf einem Feld des Teststreifens aufgenommen ist.

23. Verwendung nach einem der Ansprüche 15 bis 22, wobei das Protein Prothrombin, Faktor VII, Faktor IX, Faktor X, Nephrocalcin oder Osteocalcin ist.

**Claims**

1. A method for indirectly determining the blood clotting status having the following steps:

   a) provision of a sample of body fluid which contains a protein which can be modified by c vitamin K-dependent γ-carboxylase,

   b) measuring at least two concentrations selected from a group consisting of a first concentration (C1) of carboxylated protein, a second concentration (C2) of decarboxylated protein and a total concentration (C3) of carboxylated and decarboxylated protein, where the first concentration (C1) is measured using a first antibody (A1), the second concentration (C2) is measured using a second antibody (A2) and the third concentration (C3) is measured using a third antibody (A3),

   c) forming a first ratio (Q1) from the first and second concentration (C2)
   or
   forming a second ratio (Q2) from the third (C3) and first concentration (C1)
   or
   forming a third ratio (Q3) from the third (C3) and second concentration (C2),
   where a concentration (C1, C2, C3) which is necessary for forming the first (Q1), second (Q2) or third (Q3) ratio and is not measured in step b) is calculated in accordance with the following relation:

   $$C3 - C2 = C1$$

   and

   d) correlating the first, second or third ratio (Q1, Q2, Q3) with the blood clotting status.

2. The method as claimed in claim 1, where in step b) additionally at least a first competitor (K1) is used to measure the first concentration (C1), a second competitor (K2) is used to measure the second concentration (C2) or a third competitor (K3) is used to measure the third concentration (C3).

3. The method as claimed in claim 1 or 2, where at least one of the antibodies (A1, A2, A3) or at least one of the competitors (K1, K2, K3) is conjugated to a labeling substance, in particular an enzyme, a fluorescent dye, a quencher, a gold particle, a latex particle, a biotin, streptavidin or avidin.

4. The method as claimed in any of the preceding claims, where in place of measuring the at least two concentrations as in step b) a combined signal correlating therewith is generated and measured by using two antibodies selected from a group consisting of the first (A1), the second (A2) and the third antibody (A3) and, where appropriate, at least one of the competitors (K1, K2, K3), and is directly correlated with the blood clotting status.

5. The method as claimed in claim 4, where the combined signal is a combined color generated in particular by fluorescent dyes, a fluorescent signal elicited by the Förster effect or a reduction caused by the quencher in a fluorescent signal.

6. The method as claimed in any of the preceding claims, where the body fluid is plasma, blood, saliva, urine or the like.

7. The method as claimed in any of the preceding claims, where the measurement of the first (C1), second (C2) and/or third concentration (C3) or of the combined signal takes place by an immunological method.

8. The method as claimed in claim 7, where in the immunological method at least one of the antibodies (A1, A2, A3) is immobilized on a support, in particular a plastic, a magnetic particle, a latex particle, a gold particle, a test strip or a membrane.

9. The method as claimed in any of the preceding claims, where the first (C1), second (C2) and/or third concentration (C3) and/or the combined signal is measured by means of a color reaction or fluorescence detection.

10. The method as claimed in any of the preceding claims, where the protein which can be modified by a vitamin K-dependent γ-carboxylase is prothrombin, factor VII, factor IX, factor X, nephrocalcin or osteocalcin.

11. A kit for carrying out the method as claimed in any of the preceding claims, having a first antibody (A1) for immunological determination of a first concentration (C1) of the carboxylated form of the protein and having a second antibody (A2) for immunological determination of a second concentration (C2) of the decarboxylated form of the protein, **characterized in that** first (A1) and second antibodies (A2) is in each case conjugated to a labeling substance, where the labeling substances are selected so that they are able together to generate a combined signal.

12. A kit as claimed in claim 11, where the labeling substance is an enzyme, a fluorescent dye or a quencher.

13. A kit as claimed in claim 11 or 12, where the combined signal is a combined color, a fluorescent signal elicited by the Förster effect or a reduction caused by a quencher in a fluorescent signal.

14. A kit as claimed in any of claims 11 to 13, where the protein is prothrombin, factor VII, factor IX, factor X, nephrocalcin or osteocalcin.

15. The use of a kit comprising at least two antibodies selected from a group consisting of a first antibody (A1) for immunological determination of a first concentration (C1) of the carboxylated form of the protein, a second antibody (A2) for immunological determination of a second concentration (C2) of the decarboxylated form of the protein and a third antibody (A3) for immunological determination of a total concentration (C3) of carboxylated and decarboxylated protein, for carrying out the method as claimed in any of claims 1 to 10.

16. The use as claimed in claim 15, where additionally at least a first competitor (K1) is present for measuring the first concentration (C1), a second competitor (K2) is present for measuring the second concentration (C2) or a third competitor (K3) is present for measuring the third concentration (C3).

17. The use as claimed in claim 15 or 16, where at least one of the antibodies (A1, A2, A3) or competitors (K1, K2, K3) present is conjugated to a labeling substance, in particular an enzyme, a fluorescent dye, a quencher, a gold particle, a latex particle, biotin, streptavidin or avidin.

18. The use as claimed in any of claims 15 to 17, where the first (A1) and the second antibody (A2) are immobilized on a support, in particular a plastic, a magnetic particle, a latex particle, a gold particle, a test strip or a membrane.

19. The use as claimed in claim 18, where the support is a test strip, and the first (A1) and the second antibody (A2) are each absorbed on a separate field of the test strip.

20. The use as claimed in claim 18 or 19, where a third antibody (A3) conjugated to a labeling substance, in particular an enzyme, a fluorescent dye, a quencher, a gold particle, a latex particle, biotin, streptavidin or avidin is present.

21. The use as claimed in any of claims 15 to 20, where the third antibody (A3) is immobilized on the or another support, in particular a plastic, a magnetic particle, a latex particle, a gold particle, a test strip or a membrane.

22. The use as claimed in claim 21, where the support is the or another test strip, and the third antibody (A3) is absorbed on a field of the test strip.

23. The use as claimed in any of claims 15 to 22, where the protein is prothrombin, factor VII, factor IX, factor X, nephrocalcin or osteocalcin.

**Revendications**

1. Procédé pour la détermination indirecte de l'état de coagulation sanguine en effectuant les opérations suivantes :

   a) Mise à disposition d'un échantillon de liquide corporel lequel contient une protéine modifiable par une $\gamma$-carboxylase dépendante de la vitamine K,

   b) Détermination d'au moins deux concentrations, sélectionnées à partir d'un groupe comprenant une première concentration (C1) en protéine carboxylée, une deuxième concentration (C2) en protéine décarboxylée et une concentration totale (C3) en protéine carboxylée et décarboxylée, la première concentration (C1) étant déterminée en utilisant un premier anticorps (A1), la deuxième concentration (C2) en utilisant un deuxième anticorps (A2) et la troisième concentration (C3) en utilisant un troisième anticorps (A3),

   c) Formation d'un premier quotient (Q1) à partir de la première (C1) et de la deuxième concentration (C2)
   ou
   Formation d'un deuxième quotient (Q2) à partir de la troisième (C3) et de la première concentration (C1)
   ou
   Formation d'un troisième quotient (Q3) à partir de la troisième (C3) et de la deuxième concentration (C2),

une concentration (C1, C2, C3) requise et non déterminée à l'opération mentionnée au point b) pour la formation du premier (Q1), du deuxième (Q2) ou du troisième quotient (Q3) étant calculée selon la relation suivante :

$$C3 - C2 = C1$$

et

d) corrélation du premier, deuxième ou troisième quotient (Q1, Q2, Q3) avec l'état de coagulation sanguine.

2. Procédé selon la revendication 1, en outre, à l'opération mentionnée au point b), au moins un premier compétiteur (K1) étant utilisé pour la détermination de la première concentration (C1), un deuxième compétiteur (K2) pour la détermination de la deuxième concentration (C2) ou un troisième compétiteur (K3) pour la détermination de la troisième concentration (C3).

3. Procédé selon la revendication 1 ou 2, au moins un des anticorps (A1, A2, A3) ou au moins un des compétiteurs (K1, K2, K3) étant conjugué à une substance de marquage, en particulier, une enzyme, un colorant fluorescent, un quencher, une particule d'or, une particule de latex, de la biotine, de la streptavidine ou de l'avidine.

4. Procédé selon l'une des revendications précédentes, au lieu de la détermination d'au moins deux concentrations selon l'opération mentionnée au point b), un signal mixte corrélant étant généré et déterminé en utilisant deux anticorps sélectionnés à partir d'un groupe comprenant le premier (A1), le deuxième (A2) et le troisième anticorps (A3) et, le cas échéant, au moins un des compétiteurs (K1, K2, K3), et étant corrélé directement avec l'état de coagulation sanguine.

5. Procédé selon la revendication 4, le signal mixte étant une couleur mélangée générée par des colorants fluorescents, un signal fluorescent provoqué par l'effet Förster ou une diminution d'un signal fluorescent due au quencher.

6. Procédé selon l'une des revendications précédentes, le liquide corporel étant du plasma, du sang, de la salive, de l'urine ou similaire.

7. Procédé selon l'une des revendications précédentes, la détermination de la première (C1), de la deuxième (C2) et/ou de la troisième concentration (C3) ou du signal mixte se faisant à l'aide d'un procédé immunologique.

8. Procédé selon la revendication 7, au moins un des anticorps (A1, A2, A3) étant immobilisé sur un support, en particulier, une matière plastique, une particule magnétique, une particule de latex, une particule d'or, un papier indicateur ou une membrane lors du procédé immunologique.

9. Procédé selon l'une des revendications précédentes, la première (C1), la deuxième (C2) et/ou la troisième concentration (C3) et/ou le signal mixte étant déterminé au moyen d'une réaction colorée ou d'une détection fluorescente.

10. Procédé selon l'une des revendications précédentes, la protéine modifiable par une γ-carboxylase dépendante de la vitamine K étant de la prothrombine, facteur VII, facteur IX, facteur X, de la néphrocalcine ou ostéocalcine.

11. Kit pour l'exécution du procédé selon l'une des revendications précédentes avec un premier anticorps (A1) pour la détermination immunologique d'une première concentration (C1) de la forme carboxylée de la protéine et un deuxième anticorps (A2) pour la détermination immunologique d'une deuxième concentration (C2) de la forme décarboxylée de la protéine, **caractérisé par le fait que** le premier (A1) et le deuxième anticorps (A2) sont respectivement conjugués à une substance de marquage, les substances de marquage étant sélectionnées de façon qu'elles puissent générer conjointement un signal mixte.

12. Kit selon la revendication 11, la substance de marquage étant une enzyme, un colorant fluorescent ou un quencher.

13. Kit selon la revendication 11 ou 12, le signal mixte étant une couleur mélangée, un signal fluorescent provoqué par l'effet Fôrster ou une diminution d'un signal fluorescent due à un quencher.

14. Kit selon l'une des revendications 11 à 13, la protéine étant de la prothrombine, facteur VII, facteur IX, facteur X, de la néphrocalcine ou ostéocalcine.

15. Utilisation d'un kit contenant au moins deux anticorps, sélectionnés à partir d'un groupe comprenant un premier anticorps (A1) pour la détermination immunologique d'une première concentration (C1) de la forme carboxylée de la protéine, un deuxième anticorps (A2) pour la détermination immunologique d'une deuxième concentration (C2) de la forme décarboxylée de la protéine et un troisième anticorps (A3) pour la détermination immunologique d'une concentration totale (C3) en protéine carboxylée et décarboxylée,
pour l'exécution du procédé selon l'une des revendications 1 à 10.

16. Utilisation selon la revendication 15, en outre au moins un premier compétiteur (K1) étant compris pour la détermination de la première concentration (C1), un deuxième compétiteur (K2) pour la détermination de la deuxième concentration (C2) ou un troisième compétiteur (K3) pour la détermination de la troisième concentration (C3).

17. Utilisation selon la revendication 15 ou 16, au moins un des anticorps (A1, A2, A3) compris ou un des compétiteurs (K1, K2, K3) étant conjugué à une substance de marquage, en particulier, une enzyme, un colorant fluorescent, un quencher, une particule d'or, une particule de latex, de la biotine, de la streptavidine ou de l'avidine.

18. Utilisation selon l'une des revendications 15 à 17, le premier (A1) et le deuxième anticorps (A2) étant immobilisés sur un support, en particulier, une matière plastique, une particule magnétique, une particule de latex, une particule d'or, un papier indicateur ou une membrane.

19. Utilisation selon la revendication 18, le support étant un papier indicateur et le premier (A1) et le deuxième anticorps (A2) étant respectivement sur une zone séparée du papier indicateur.

20. Utilisation selon la revendication 18 ou 19, un troisième anticorps (A3) conjugué à une substance de marquage, en particulier, une enzyme, un colorant fluorescent, un quencher, une particule d'or, une particule de latex, de la biotine, de la streptavidine ou de l'avidine, étant contenu.

21. Utilisation selon l'une des revendications 15 à 20, le troisième anticorps (A3) étant immobilisé sur le ou un autre support, en particulier, une matière plastique, une particule magnétique, une particule de latex, une particule d'or, un papier indicateur ou une membrane.

22. Utilisation selon la revendication 21, le support étant le ou un autre papier indicateur et le troisième anticorps (A3) étant logé sur une zone du papier indicateur.

23. Utilisation selon l'une des revendications 15 à 22, la protéine étant de la prothrombine, facteur VII, facteur IX, facteur X, de la néphrocalcine ou ostéocalcine.

*Fig. 1*

Fig. 2

Fig. 3